(19) Europäisches Patentamt
European Patent Office
Office européen des brevets

(11) EP 2 694 023 B1

# (12) EUROPEAN PATENT SPECIFICATION

(45) Date of publication and mention of the grant of the patent:
**18.05.2016 Bulletin 2016/20**

(21) Application number: **12710971.8**

(22) Date of filing: **29.03.2012**

(51) Int Cl.:
***A61K 8/37*** *(2006.01)* ***A61Q 5/00*** *(2006.01)*

(86) International application number:
**PCT/EP2012/055658**

(87) International publication number:
**WO 2012/130954 (04.10.2012 Gazette 2012/40)**

## (54) COSMETIC COMPOSITION COMPRISING 4-(3-ETHOXY-4-HYDROXYPHENYL)-2-BUTANONE AND A LIPOPHILIC SOLVENT

KOSMETISCHES MITTLE MIT 4-(3-ETHOXY-4-HYDROXYPHENYL)-2-BUTANON UND EINEN LIPOPHILE LÖSUNGSMITTEL

COMPOSITIONS COSMETIQUES CONTENANT DE LA 4-(3-ETHOXY-4-HYDROXYPHENYL)-2-BUTANONE ET UN SOLVANT LIPOPHILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2011 FR 1152788**
**22.06.2011 US 201161499707 P**

(43) Date of publication of application:
**12.02.2014 Bulletin 2014/07**

(73) Proprietor: **L'Oréal**
**75008 Paris (FR)**

(72) Inventors:
- **CHEVALIER, Véronique**
**F-94440 Villecresnes (FR)**
- **OUATTARA, Sofiane**
**F-94400 Vitry-sur-Seine (FR)**

(74) Representative: **Le Blainvaux Bellegarde, Françoise**
**L'Oréal**
**D.I.P.I.**
**25-29 Quai Aulagnier**
**92665 Asnières-sur-Seine Cedex (FR)**

(56) References cited:
**EP-A1- 0 334 967** **WO-A1-2011/039445**
**WO-A1-2011/042358** **US-A1- 2004 156 799**

- **BRANDRUP J ET AL: "Polymer Handbook, Chapter VII, Ed. 3", 1 January 1989 (1989-01-01), POLYMER HANDBOOK, NEW YORK, WILEY & SONS.; US, PAGE(S) 526 - 557, XP002476719, table 3.1**
- **GRULKE ERIC A ED - BRANDRUP J ET AL: "Polymer Handbook, Chapter VII - Solubility Parameter Values", POLYMER HANDBOOK; [A WILEY-INTERSCIENCE PUBLICATION], NEW YORK, NY : WILEY, US, 1 January 1999 (1999-01-01), pages 675-688, XP002439625, ISBN: 978-0-471-16628-3**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a cosmetic composition containing 4-(3-ethoxy-4-hydroxyphenyl)-2-butanone and a particular organic solvent. 4-(3-Ethoxy-4-hydroxyphenyl)-2-butanone is an interesting substance as a preserving agent for cosmetic compositions, for protecting the compositions against microbial contamination.

However, this compound, which is in solid form at room temperature, is very sparingly soluble in water or glycerol, or alternatively in 2-octyldodecanol.

Now, it is necessary for this compound to be formulated in a solubilized form in order to fully exploit its activity, and it is also preferable for its solubilization to be maintained over time in order to avoid any recrystallization during storage of compositions comprising such a compound.

The object of the present invention is, precisely, to propose a novel galenical formulation of 4-(3-ethoxy-4-hydroxyphenyl)-2-butanone that overcomes the abovementioned drawbacks, and thus enables this compound to be incorporated in a long-lasting solubilized form.

Specifically, the inventors have discovered, unexpectedly, that the combination of 4-(3-ethoxy-4-hydroxyphenyl)-2-butanone with at least one particular organic solvent enables this compound to be solubilized while avoiding its recrystallization, especially after storage for two months at room temperature (25°C).

More precisely, a subject of the invention is a composition, especially a cosmetic composition, comprising, in a physiologically acceptable oily medium, 4-hydroxyphenyl)-2-butanone and an organic solvent with solubility parameters in the Hansen solubility space such that $4.5 < \delta_a < 7$ and $14 < \delta_d < 22$, chosen from isopropyl N-lauroyl sarcosinate, capric/caprylic acid triglycerides, 2-phenylethyl benzoate, isopropyl palmitate and isopropyl myristate.

A further subject of the invention is a process for the non-therapeutic cosmetic treatment of keratin materials, comprising the application to the keratin materials of a composition as described previously. The process may be a cosmetic process for caring for, making up or cleansing keratin materials.

As specified hereinbelow, 4-(3-ethoxy-4-hydroxyphenyl)-2-butanone is advantageously present in the compositions in accordance with the invention in a solubilized form.

4-(3-Ethoxy-4-hydroxyphenyl)-2-butanone is a compound of formula:

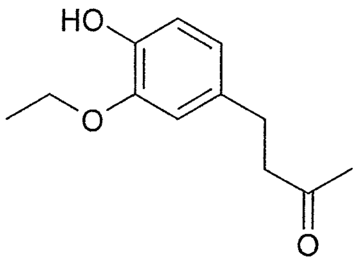

The compound 4-(3-ethoxy-4-hydroxyphenyl)-2-butanone may be present in the composition according to the invention in a content ranging from 0.01% to 10% by weight, better still from 0.1% to 7% by weight, from 0.5% to 7% by weight, from 0.75% to 5% by weight and preferably ranging from 1% to 5% by weight, relative to the total weight of the composition.

The composition according to the invention comprises an effective amount of at least one organic solvent with solubility parameters in the Hansen solubility space such that $4.5 < \delta_a < 7$ and $14 < \delta_d < 22$, chosen from isopropyl N-lauroyl sarcosinate, capric/caprylic acid triglycerides, 2-phenylethyl benzoate, isopropyl palmitate and isopropyl myristate.

The global solubility parameter δ according to the Hansen solubility space is defined in the article "Solubility parameter values" by Eric A. Grulke in the book "Polymer Handbook", 3rd Edition, Chapter VII, pp. 519-559, by the relationship:

$$\delta = (\delta^2 = \delta_d^2 + \delta_p^2 + \delta_h^2)^{1/2}$$

in which:

- $\delta_d$ characterizes the London dispersion forces derived from the formation of dipoles induced during molecular impacts,
- $\delta_p$ characterizes the Debye interaction forces between permanent dipoles, and
- $\delta_h$ characterizes the specific interaction forces (such as hydrogen bonding, acid/base, donor/acceptor, etc.). The

definition of solvents in the Hansen three-dimensional solubility space is described in the article by C.M. Hansen: "The three-dimensional solubility parameters", J. Paint Technol. 39, 105 (1967).

The parameter $\delta_a$ is defined by the following relationship: $\delta_a^2 = \delta_p^2 + \delta_h^2 = \delta^2 - \delta_d^2$

The parameters $\delta_d$, $\delta_p$, $\delta_h$ and $\delta_a$ are expressed in $(J/cm^3)^{1/2}$.

Preferably, the organic solvent has solubility parameters such that $4.5<\delta_a<7$ and $14<\delta_d<17$

Preferentially, the organic solvent has solubility parameters such that $6<\delta_a<7$ and $16<\delta_d<17$

The organic solvent used according to the invention may be chosen from isopropyl N-lauroyl sarcosinate ($\delta_a$ = 4.91; $\delta_d$ = 14.9), capric/caprylic acid triglycerides ($\delta_a$ = 6.7; $\delta_d$ = 16.60) such as the product sold by the company Stéarineries Dubois or the product sold under the name Miglyol 812 N by the company Sasol; 2-phenylethyl benzoate (X-Tend 226 from ISP) ($\delta_a$ = 6.36; $\delta_d$ = 21.48), isopropyl palmitate ($\delta_a$ = 4.74; $\delta_d$ = 16.10), isopropyl myristate ($\delta_a$ = 5.00; $\delta_d$ = 16.00).

Preferably, the organic solvent is chosen from isopropyl N-lauroyl sarcosinate, capric/caprylic acid triglycerides (60/40); 2-phenylethyl benzoate (X-Tend 226 from ISP).

Preferentially, the organic solvent is chosen from isopropyl N-lauroyl sarcosinate and capric/caprylic acid triglycerides.

The solvent may be present in the composition according to the invention in a content ranging from 0.05% to 30% by weight, better still from 0.05% to 10% by weight, preferably ranging from 0.1 % to 5% by weight and preferentially ranging from 0.1 % to 2.5% by weight, relative to the total weight of the composition.

According to one embodiment, the organic solvent in accordance with the invention and the 4-(3-ethoxy-4-hydroxyphenyl)-2-butanone (referred to as the compound) may be present in a solvent/compound mass ratio of less than or equal to 10, especially less than or equal to 5, better still less than or equal to 4, especially ranging from 0.5 to 4 and preferably ranging from 0.5 to 1.5.

The compositions used according to the invention contain a physiologically acceptable medium, i.e. a medium that is compatible with human keratin materials such as the skin, the scalp, the hair and the nails.

The composition may comprise at least one additional oil. As oils that may be used, examples that may be mentioned include:

- hydrocarbon-based oils of plant origin, such as liquid triglycerides of fatty acids containing from 4 to 10 carbon atoms, for instance heptanoic or octanoic acid triglycerides, or alternatively, for example, sunflower oil, maize oil, soybean oil, marrow oil, grapeseed oil, sesame seed oil, hazelnut oil, apricot oil, macadamia oil, arara oil, castor oil, avocado oil, jojoba oil and shea butter oil;
- synthetic esters and ethers, especially of fatty acids, for instance the oils of formulae $R_1COOR_2$ and $R_1OR_2$ in which $R_1$ represents a fatty acid residue containing from 8 to 29 carbon atoms and $R_2$ represents a branched or unbranched hydrocarbon-based chain containing from 3 to 30 carbon atoms, for instance Purcellin oil, isononyl isononanoate, 2-ethylhexyl palmitate, 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate or triisocetyl citrate; fatty alcohol heptanoates, octanoates or decanoates; polyol esters, for instance propylene glycol dioctanoate, neopentyl glycol diheptanoate and diethylene glycol diisononanoate; and pentaerythritol esters, for instance pentaerythrityl tetraisostearate;

- linear or branched hydrocarbons of mineral or synthetic origin, such as volatile or non-volatile liquid paraffins, and derivatives thereof, petroleum jelly, polydecenes, and hydrogenated polyisobutene such as Parleam oil;
- fatty alcohols containing from 8 to 26 carbon atoms, for instance cetyl alcohol, stearyl alcohol and a mixture thereof (cetylstearyl alcohol), octyldodecanol, 2-butyloctanol, 2-hexyldecanol, 2-undecylpentadecanol, oleyl alcohol or linoleyl alcohol;
- silicone oils, for instance volatile or non-volatile polymethylsiloxanes (PDMS) with a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end of a silicone chain, these groups containing from 2 to 24 carbon atoms; phenylsilicones, for instance phenyl trimethicones, phenyl dimethicones, phenyltrimethylsiloxydiphenylsiloxanes, diphenyl dimethicones, diphenylmethyldiphenyltrisiloxanes or 2-phenylethyl trimethylsiloxy silicates, and polymethylphenylsiloxanes;
- mixtures thereof.

In the list of oils mentioned above, the term "hydrocarbon-based oil" means any oil mainly comprising carbon and hydrogen atoms, and possibly ester, ether, fluoro, carboxylic acid and/or alcohol groups.

The composition according to the invention may comprise substances that are solid at room temperature (25°C), for instance fatty acids comprising from 8 to 30 carbon atoms, such as stearic acid, lauric acid, palmitic acid and oleic

acid; waxes such as lanolin, beeswax, carnauba wax or candelilla wax, paraffin waxes, microcrystalline waxes, ceresin or ozokerite, and synthetic waxes such as polyethylene waxes and Fischer-Tropsch waxes.

These fatty substances may be chosen in a varied manner by a person skilled in the art so as to prepare a composition having the desired properties, for example in terms of consistency or texture.

The composition according to the invention may comprise an aqueous phase.

The composition may comprise water, which may be present in a content ranging from 5% to 80% by weight and preferably ranging from 35% to 75% by weight relative to the total weight of the composition.

The composition may also comprise a polyol that is water-miscible at room temperature (25°C) chosen especially from polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, dipropylene glycol and diethylene glycol. This polyol is different from the organic solvents in accordance with the invention.

The compositions according to the invention may be in the form of oil-in-water (O/W) emulsions, water-in-oil (W/O) emulsions or multiple emulsions (triple: W/O/W or O/W/O), oily solutions or oily gels. These compositions are prepared according to the usual methods.

According to one particular embodiment of the invention, the composition according to the invention is a water-in-oil (W/O) or oil-in-water (O/W) emulsion. The proportion of the oily phase of the emulsion may range from 5% to 80% by weight and preferably from 5% to 50% by weight relative to the total weight of the composition.

The emulsions generally contain at least one emulsifier chosen especially from amphoteric, anionic, cationic and nonionic emulsifiers, used alone or as a mixture, and optionally a co-emulsifier. The emulsifiers are appropriately chosen according to the emulsion to be obtained (W/O or O/W). The emulsifier and the co-emulsifier are generally present in the composition in a proportion ranging from 0.3% to 30% by weight and preferably from 0.5% to 20% by weight relative to the total weight of the composition.

For W/O emulsions, examples of emulsifiers that may be mentioned include dimethicone copolyols, such as the mixture of cyclomethicone and dimethicone copolyol sold under the trade name DC 5225 C by the company Dow Corning, and alkyl dimethicone copolyols such as the lauryl dimethicone copolyol sold under the name Dow Corning 5200 Formulation Aid by the company Dow Corning, and the cetyl dimethicone copolyol sold under the name Abil EM 90° by the company Goldschmidt. A crosslinked elastomeric solid organopolysiloxane comprising at least one oxyalkylene group, such as those obtained according to the procedure of Examples 3, 4 and 8 of patent US-A-5 412 004 and of the examples of patent US-A-5 811 487, especially the product of Example 3 (synthesis example) of patent US-A-5 412 004, such as the product sold under the reference KSG 21 by the company Shin-Etsu, may also be used as surfactants for W/O emulsions.

For O/W emulsions, examples of emulsifiers that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alkyl ethers; sugar esters such as sucrose stearate; and mixtures thereof, such as the mixture of glyceryl stearate and PEG-40 stearate (polyethylene glycol stearate containing 40 ethylene glycol units).

In a known manner, the composition according to the invention may also contain adjuvants that are common in cosmetics or dermatology, such as gelling agents, film-forming polymers, preserving agents, fragrances, fillers, UV-screening agents, bactericides, odour absorbers, dyestuffs, plant extracts, cosmetic and dermatological active agents, and salts. The amounts of these various adjuvants are those conventionally used in the field under consideration, for example from 0.01 % to 20% of the total weight of the composition.

The invention is illustrated in greater detail in the example that follows. The amounts of the ingredients are expressed as weight percentages.

## Example 1: Solubility test

The solubility tests were performed with 4-(3-ethoxy-4-hydroxyphenyl)-2-butanone.

The test solvent was poured into a beaker, an amount of the compound was added with magnetic stirring and the mixture was left to stand for between 1 hour and 24 hours. The mixture was then heated to 50°C and then cooled to room temperature (25°C) over 24 hours. The system was then observed to see if the amount of compound introduced recrystallized or remained dissolved.

The amount of compound that can be dissolved in the test solvent was determined in this manner. The maximum value corresponds to the amount at and above which the ester compound stops dissolving in the evaluated solvent.

The following results were obtained in several evaluated solvents:

| Solvent | Degree of solubilization (weight%) |
|---|---|
| Water* | < 1 |
| 2-Ethylhexyl palmitate* | 10 |
| 2-Octyldodecanol* | 10 |
| Isopropyl N-lauroyl sarcosinate | > 20 |
| Capric/caprylic acid triglycerides (1) | > 20 |
| 2-Phenylethyl benzoate | > 20 |
| * solvent not forming part of the invention<br>(1) 70/30 C8-C10 Triglycerides (Dub MCT 7030) from Stéarineries Dubois | |

It was thus found that the solvents such as 2-phenylethyl benzoate, isopropyl N-lauroyl sarcosinate and capric/caprylic acid triglycerides dissolved the active agent, whereas 2-ethylhexyl palmitate ($\delta_{a\,=}$ 4.23; $\delta_{d\,=}$ 16.20) and 2-octyldodecanol ($\delta_a$ = 7.70; $\delta_d$ = 16.40) dissolved the active agent less well.

**Example 2:**

A facial care cream (oil-in-water emulsion) having the following composition was prepared:

| | |
|---|---|
| Xanthan gum (Rhodicare XC from Rhodia) | 0.15 |
| Mixture of glyceryl stearate and PEG-100 stearate (Arlacel® 165 FL from Uniqema) | 2.5 |
| 1,3-Butylene glycol | 0.525 |
| Stearyl alcohol | 1 |
| Stearic acid | 1.2 |
| Capric/caprylic acid triglycerides (70/30 C8-C10 Triglycerides (Dub MCT 7030) from Stéarineries Dubois) | 5 |
| Acrylamide/sodium acrylamido-2-methylpropanesulfonate copolymer as a reverse emulsion at 40% in isoparaffin/water (Sepigel 305 from SEPPIC) | 1.7 |
| Sodium hyaluronate (Cristalhyal from Soliance) | 0.05 |
| 4-(3-Ethoxy-4-hydroxyphenyl)-2-butanone | 2 |
| Glycerol | 5 |
| Cetyl alcohol | 1 |
| Tocopheryl acetate | 0.2 |
| Cyclopentasiloxane | 5 |
| Water | qs 100 |

The composition is stable and homogeneous after storage for 2 months at 45°C.

**Example 3:**

A body milk having the following composition was prepared:

| | |
|---|---|
| 2-Ethylhexyl palmitate | 7 |
| Citric acid | 0.06 |
| Glycerol | 3 |
| Xanthan gum (Keltrol CG-T from CP Kelco) | 0.1 |
| Polymerized acrylic acid/stearyl methacrylate copolymer in an ethyl acetate/cyclohexane mixture (Pemulen TR-1 Polymer from Noveon) | 0.1 |
| 4-(3-Ethoxy-4-hydroxyphenyl)-2-butanone | 2 |
| Mixture of corn oil, rice bran oil, sesame oil and wheatgerm oil (29/40/20/10) | 5 |
| Triethanolamine | 0.4 |

(continued)

| | |
|---|---|
| Carboxyvinyl polymer (Synthalen K from 3V) | 0.3 |
| Acrylamide/sodium acrylamido-2-methylpropanesulfonate copolymer as a reverse emulsion at 40% in isoparaffin/water (Sepigel 305 from SEPPIC) | 0.5 |
| Water qs | 100 |

The composition is stable and homogeneous after storage for 2 months at 45°C.

**Example 4:**

A facial care cream (water-in-oil emulsion) having the following composition was prepared:

| | |
|---|---|
| Ethanol | 5 |
| Polydimethylsiloxane 10 cSt | 6.75 |
| Mixture of acetylated ethylene glycol stearate and glyceryl tristearate (Unitwix from United Guardian) | 0.5 |
| Aluminium salt of corn starch esterified with octenylsuccinic anhydride (Dry Flo Plus 28-1160 from National Starch) | 3 |
| Liquid fraction of shea butter (Shea Olein from Olvea) | 4 |
| Polyglyceryl-4 isostearate (GI 34 from Goldschmidt) | 0.5 |
| 4-(3-Ethoxy-4-hydroxyphenyl)-2-butanone | 2 |
| Apricot kernel oil | 1.4 |
| Vinylidene chloride/acrylonitrile/methyl methacrylate copolymer microspheres expanded with isobutane (Expancel 551 DE 40 D42 from Expancel) | 0.1 |
| Magnesium sulfate | 0.7 |
| Hydrogenated polyisobutene (Parleam from NOF Corporation) Capric/caprylic acid triglycerides (70/30 C8-C10 Triglycerides (Dub MCT 7030) | 10 |
| from Stéarineries Dubois) | 5 |
| Cetyl dimethicone copolyol (Abil EM 90 from Goldschmidt) | 1.5 |
| Water qs | 100 |

The composition is stable and homogeneous after storage for 2 months at 45°C.

## Claims

1. Composition comprising, in a physiologically acceptable oily medium, 4-(3-ethoxy-4-hydroxyphenyl)-2-butanone and an organic solvent with solubility parameters in the Hansen solubility space such that $4.5 < \delta_a < 7$ and $14 < \delta_d < 22$, chosen from isopropyl N-lauroyl sarcosinate, capric/caprylic acid triglycerides, 2-phenylethyl benzoate, isopropyl palmitate and isopropyl myristate.

2. Composition according to either of the preceding claims, **characterized in that** the organic solvent is chosen from isopropyl N-lauroyl sarcosinate and capric/caprylic acid triglycerides.

3. Composition according to any one of the preceding claims, **characterized in that** the organic solvent is present in a content ranging from 0.05% to 30% by weight, better still from 0.05% to 10% by weight, preferably ranging from 0.1 % to 5% by weight and preferentially ranging from 0.1% to 2.5% by weight relative to the total weight of the composition.

4. Composition according to any one of the preceding claims, **characterized in that** the 4-(3-ethoxy-4-hydroxyphenyl)-2-butanone is present in a content ranging from 0.01% to 10% by weight, better still from 0.1 % to 7% by weight, from 0.5% to 7% by weight, from 0.75% to 5% by weight and preferably ranging from 1% to 5% by weight, relative to the total weight of the composition.

5. Composition according to any one of the preceding claims, **characterized in that** the said organic solvent and the 4-(3-ethoxy-4-hydroxyphenyl)-2-butanone are present in an organic solvent/4-(3-ethoxy-4-hydroxyphenyl)-2-butanone mass ratio of less than or equal to 10, especially less than or equal to 5 and better still less than or equal to 4, especially ranging from 0.5 to 4 and preferably ranging from 0.5 to 1.5.

6. Composition according to any one of the preceding claims, **characterized in that** it comprises an additional oil.

7. Composition according to any one of the preceding claims, **characterized in that** it is in the form of a water-in-oil or oil-in-water emulsion, preferably an oil-in-water emulsion.

8. Non-therapeutic cosmetic treatment process for caring for and/or making up and/or cleansing keratin materials, comprising the application to the said keratin materials of a composition according to any one of the preceding claims.

## Patentansprüche

1. Zusammensetzung, die in einem physiologisch unbedenklichen öligen Medium 4-(3-Ethoxy-4-hydroxyphenyl)-2-butanon und ein organisches Lösungsmittel mit solchen Löslichkeitsparametern im Löslichkeitsraum nach Hansen, dass $4{,}5 < \delta_a < 7$ und $14 < \delta_d < 22$, das aus Isopropyl-N-lauroyl-sarkosinat, Capryl-/Caprinsäuretriglyceriden, 2-Phenylethylbenzoat, Isopropylpalmitat und Iso-propylmyristat ausgewählt ist, umfasst.

2. Zusammensetzung nach einem der vorhergehenden Ansprüche, **dadurch gekennzeichnet, dass** das organische Lösungsmittel aus Isopropyl-N-lauroyl-sarkosinat und Capryl-/Caprinsäuretriglyceriden ausgewählt ist.

3. Zusammensetzung nach einem der vorhergehenden Ansprüche, **dadurch gekennzeichnet, dass** das organische Lösungsmittel in einem Gehalt im Bereich von 0,05 bis 30 Gew.-%, noch besser 0,05 bis 10 Gew.-%, vorzugsweise im Bereich von 0,1 bis 5 Gew.-% und bevorzugt im Bereich von 0,1 bis 2,5 Gew.-%, bezogen auf das Gesamtgewicht der Zusammensetzung, vorliegt.

4. Zusammensetzung nach einem der vorhergehenden Ansprüche, **dadurch gekennzeichnet, dass** das 4-(3-Ethoxy-4-hydroxyphenyl)-2-butanon in einem Gehalt im Bereich von 0,01 bis 10 Gew.-%, noch besser 0,1 bis 7 Gew.-%, 0,5 bis 7 Gew.-%, 0,75 bis 5 Gew.-% und vorzugsweise im Bereich von 1 bis 5 Gew.-%, bezogen auf das Gesamtgewicht der Zusammensetzung, vorliegt.

5. Zusammensetzung nach einem der vorhergehenden Ansprüche, **dadurch gekennzeichnet, dass** das organische Lösungsmittel und das 4-(3-Ethoxy-4-hydroxyphenyl)-2-butanon in einem Massenverhältnis von organischem Lösungsmittel zu 4-(3-Ethoxy-4-hydroxyphenyl) -2-butanon kleiner gleich 10, speziell kleiner gleich 5 und noch besser kleiner gleich 4, speziell im Bereich von 0,5 bis 4 und vorzugsweise im Bereich von 0,5 bis 1,5 vorliegen.

6. Zusammensetzung nach einem der vorhergehenden Ansprüche, **dadurch gekennzeichnet, dass** sie ein zusätzliches Öl umfasst.

7. Zusammensetzung nach einem der vorhergehenden Ansprüche, **dadurch gekennzeichnet, dass** sie in Form einer Wasser-in-Öl- oder Öl-in-Wasser-Emulsion, vorzugsweise einer Öl-in-Wasser-Emulsion, vorliegt.

8. Nichttherapeutisches kosmetisches Behandlungsverfahren zum Pflegen und/oder Schminken und/oder Reinigen von Keratinmaterialien, bei dem man auf die Keratinmaterialien eine Zusammensetzung nach einem der vorhergehenden Ansprüche aufbringt.

## Revendications

1. Composition comprenant, dans un milieu huileux physiologiquement acceptable, de la 4-(3-éthoxy-4-hydroxyphényl)butan-2-one et un solvant organique ayant des paramètres de solubilité selon l’espace de solubilité de Hansen tels que $4{,}5 < \delta_a < 7$ et $14 < \delta_d < 22$, choisi parmi le N-lauroyl sarcosinate d’isopropyle, les triglycérides d’acide caprique/caprylique, le 2-phényl éthyl ester de l’acide benzoïque, le palmitate d’isopropyle, le myristate d’isopropyle.

2. Composition selon l’une quelconque des revendications précédentes, **caractérisée en ce que** le solvant organique est choisi parmi le N-lauroyl sarcosinate d’isopropyle et les triglycérides d’acide caprique/caprylique.

3. Composition selon l'une quelconque des revendications précédentes, **caractérisée en ce que** le solvant organique est présent en une teneur allant de 0,05% à 30% en poids, mieux de 0,05% à 10% en poids, de préférence allant de 0,1 % à 5 % en poids, et préférentiellement allant de 0,1 % à 2,5 % en poids par rapport au poids total de la composition.

4. Composition selon l'une quelconque des revendications prévédentes, **caractérisée en ce que** la 4-(3-éthoxy-4-hydroxyphényl)butan-2-one est présente en une teneur allant de 0,01 % à 10 % en poids, mieux de 0,1% à 7% en poids, de 0,5 % à 7 % en poids, de 0,75 % à 5 % en poids et de préférence allant de 1 % à 5 % en poids par rapport au poids total de la composition.

5. Composition selon l'une quelconque des revendications précédentes, **caractérisée en ce que** le solvant organique et la 4-(3-éthoxy-4-hydroxyphényl)butan-2-one sont présents en un rapport massique solvant organique / 4-(3-éthoxy-4-hydroxyphényl)butan-2-one inférieur ou égal à 10, notamment inférieur ou égal à 5, mieux inférieur ou égal à 4, notamment allant de 0,5 à 4, et de préférence allant de 0,5 à 1,5.

6. Composition selon l'une quelconque des revendications précédentes, **caractérisée en ce qu'**elle comprend une huile additionnelle.

7. Composition selon l'une quelconque des revendications précédentes, **caractérisée en ce qu'**elle se présente sous la forme d'une émulsion eau-dans-huile ou huile-dans-eau, de préférence une émulsion huile-dans-eau.

8. Procédé de traitement cosmétique non thérapeutique de soin et/ou de maquillage et/ou de nettoyage des matières kératiniques comprenant l'application sur lesdites matières kératiniques d'une composition selon l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5412004 A
- US 5811487 A


### Non-patent literature cited in the description


- Solubility parameter values. **ERIC A. GRULKE.** Polymer Handbook. 519-559
- **C.M. HANSEN.** The three-dimensional solubility parameters. *J. Paint Technol.,* 1967, vol. 39, 105